# FASCICULE DE BREVET EUROPEEN

(11) **EP 3 060 058 B1**
(45) Date de publication et mention de la délivrance du brevet: **15.05.2019**
(21) Numéro de dépôt: 14824878.4
(22) Date de dépôt: 22.10.2014
(51) Int. Cl.: A01N 63/00, A01P 1/00

(54) **PROCÉDÉ DE LUTTE BIOLOGIQUE CONTRE NAEGLERIA FOWLERI, ET AGENT DÉSINFECTANT CONTENANT DES PROTOZOAIRES DE L'ESPÈCE WILLAERTIA MAGNA**
BIOLOGISCHES BEKÄMPFUNGSVERFAHREN GEGEN NAEGLERIA FOWLERI UND DESINFIZIERENDES MITTEL ENTHALTEND PROTOZOEN DER SPEZIES WILLAERTIA MAGNA
PROCESS FOR THE BIOLOGICAL CONTROL OF NAEGLERIA FOWLERI AND DISINFECTING PRODUCT CONTAINING PROTOZOANS OF THE SPECIES WILLAERTIA MAGNA

(30) Priorité: 23.10.2013 FR 1360347
(43) Date de publication de la demande: 31.08.2016
(73) Titulaire: Amoeba, 69680 Chassieu (FR)
(72) Inventeur: PLASSON, Fabrice, 69003 Lyon (FR); MAMERI, Mouh Oulhadj, 69120 Vaulx en Velin (FR)
(74) Mandataire: Cabinet Plasseraud
(86) Numéro de dépôt international: PCT/FR2014/052691
(87) Numéro de publication internationale: WO 2015/059411

(56) Documents cités:
- WO-A2-2008/043969
- US-A- 5 344 838

## Description

La présente invention concerne un nouveau procédé de lutte biologique contre la présence du genre *Naegleria* et en particulier de l'espèce *Naegleria fowleri,* et sa prolifération.
*Naegleria fowleri (N.f.)* est une amibe libre appartenant à la famille des *Vahlkampfiidaes.* Cette amibe est responsable chez l'homme d'une gravissime pathologie, fort heureusement extrêmement rare (235 cas décelés en 2007) : la méningo-encéphalite amibienne primitive (MEAP) (Cervantes-Sandoval I, 2008 ;Kemble SK, 2012 ; TW, 2010 ; Su MY, 2013). L'infection par ces amibes libres est de pronostic catastrophique en une semaine environ et de quelques semaines sous traitement antibiotique (Su MY, 2013). Il n'existe pas de traitement véritablement efficace contre cette infection, fort heureusement la maladie est rare et demande que des conditions spécifiques soient réunies pour déclencher la MEAP. Ces conditions particulières ainsi que l'inoculum requis sont encore méconnues à l'heure actuelle. Cependant certains médicaments et antibiotiques semblent influer sur l'évolution de l'infection comme l'amphotéricine B, la rifampicine et la miconazole qui associés ont prouvés leur efficacité sur 2 ou 3 personnes. En 1992, la littérature ne rapportait que 7 cas de survie avérée à la MEAP (Gautam PL, 2012) et uniquement chez des sujets très jeunes de 2 à 14 ans et dont le traitement et l'infection ont laissé des séquelles neurologiques plus ou moins importantes. Le taux de survie est donc encore plus bas que pour l'infection au virus Ebola. Aussi, la surveillance et le contrôle de cette amibe libre constituent une préoccupation de plus en plus importante.

De façon générale, il est connu que dans l'environnement, *Naegleria fowleri* présente une répartition ubiquitaire (Martinez AJ, 1997), puisque cette amibe a été isolée du sol, des eaux de rivière et de lac (Jamerson M, 2009) ou des eaux de rejets industriels, et des biofilms (Goudot S, 2012 ; S.A. Huws, 2005), caractéristiques qu'elle partage avec d'autres amibes libres. Plusieurs bactéries potentiellement pathogènes dont *Legionella pneumophila,* ont développé des mécanismes pour survivre et se répliquer à l'intérieur des amibes libres (Huang SW, 2010 ; De Jonckheere, 2011). De plus, il a été démontré que les centrales nucléaires participaient fortement au développement de l'amibe *Naegleria fowleri* par un réchauffement de plusieurs degrés de l'eau de rivière. En effet, l'amibe *Naegleria fowleri* est thermophile avec des gammes de températures de développement allant de 25°C à 45°C (Visvesvara GS, 2007).

EDF exploitant plus de 11 centrales nucléaires en France a quantifié le risque relatif au taux de *Naegleria fowleri* détecté dans les eaux.

Pour mieux appréhender ce risque, la Direction des Etudes et Recherches et le Service des Etudes Médicales d'EDF ont calculé les risques de décès par MEAP lors d'une baignade, en fonction de la concentration en *Naegleria fowleri* dans l'eau. Ce risque peut se décomposer de la façon suivante :
risque pour 1 baignade = probabilité d'inhaler "n" *Naegleria fowleri* lorsque l'on se baigne dans une eau où les *Naegleria fowleri* sont présentes à la concentration "c" (10 ml d'eau inhalée par baignade)
multiplié par
   probabilité de décès lorsque l'on a inhalé "n" *Naegleria fowleri* (modélisation d'après les données animales).

En choisissant le modèle log normal, qui donne les estimations les plus basses, et qui est en bonne adéquation avec les données réelles (USA, Australie, Nouvelle-Zélande), on obtient les risques suivants :

### Concentration Risque pour une quantité n de N.f. dans l'eau de baignade

1 *Naegleria fowleri* / litre, risque = 10⁻⁸ soit un décès pour 100 millions de baignades
10 *Naegleria fowleri* / litre, risque = 1,45 x 10⁻⁷ soit un décès pour 7 millions de baignades
100 *Naegleria fowleri* / litre, risque = 7,24 x 10⁻⁶ soit un décès pour 140 000 baignades
1000 *Naegleria fowleri* / litre, risque = 1,34 x 10⁻³ soit un décès pour 746 baignades
Conformément aux recommandations du Conseil supérieur d'hygiène publique de France (CSHPF), le dépassement de la valeur limite de 100 *Naegleria fowleri* (N.f.) par litre doit conduire à une interdiction de la pratique de la baignade (cf. notamment avis du CSHPF du 4 mai 2004 relatif au retour d'expérience des traitements anti-amibiens à la monochloramine réalisés en 2003 par EDF sur les centrales nucléaires de production d'électricité (CNPE) de Bugey, Chooz, Dampierre, Golfech et Nogent).

Dans ce contexte, les inventeurs ont mis en évidence, de façon totalement inattendue, que des protozoaires de l'espèce Willaertia magna correspondant à la souche déposée sous le numéro PTA 7824 à l'ATCC ou à la souche déposée sous le numéro PTA 7825 à l'ATCC éradique les amibes libres *Naegleria fowleri.* Cet effet biocide est doublé de la capacité de prédation déjà démontrée de *Willaertia magna* envers d'autres agents bactériens tels que les bactéries *legionella pneumophila*, *Pseudomonas* et *Listéria* (Bodennec Jacques 2006).

La présente invention a donc tout d'abord pour objet un procédé de lutte contre la prolifération des *Naegleria,* en particulier *Naegleria fowleri,* qui utilise des protozoaires de l'espèce Willaertia magna correspondant à la souche déposée sous le numéro PTA 7824 à l'ATCC ou à la souche déposée sous le numéro PTA 7825 à l'ATCC. Les procédés conformes à l'invention n'incluent pas les méthodes de traitement appliquées au corps humain ou animal. Dans le procédé selon l'invention, c'est le plus souvent un flux gazeux ou liquide qui est traité avec des protozoaires de l'espèce Willaertia magna correspondant à la souche déposée sous le numéro PTA 7824 à l'ATCC ou à la souche déposée sous le numéro PTA 7825 à l'ATCC. Mais il peut s'agir aussi d'une surface solide.

Le procédé selon l'invention trouve, en particulier application, dans la désinfection des réseaux de distribution d'eaux sanitaires ou d'eaux industrielles, des circuits de refroidissement par exemple de type nucléaire, des installations industrielles, ou des réseaux de climatisation. Il peut être mise en oeuvre pour lutter contre la formation de biofilms dans les canalisations d'eau, les surfaces en contact ou non avec des denrées alimentaires humaines ou animales.

Les protozoaires pourront être directement ajoutés aux eaux ou aux liquides circulant dans les canalisations ou dans les réseaux à traiter. Il est également possible de les pulvériser, par exemple sous la forme d'une solution aqueuse en aérosol, dans les réseaux, les cheminées, les installations et les surfaces industrielles à désinfecter.

Les protozoaires utilisés dans le cadre de l'invention correspondent à la souche déposée le 21 août 2006 sous le numéro **PTA 7824** à l'ATCC ou à la souche déposée le 21 août 2006 sous le numéro **PTA 7825** à

l'ATCC, ces deux souches ayant été déposées aux noms du CENTRE NATIONAL DE LA RECHERCHE SCIENTIFIQUE (CNRS) - 3 rue Michel Ange - 75794 PARIS CEDEX 16 / France et UNIVERSITE LYON 1 CLAUDE BERNARD - 43 Boulevard du 11 Novembre 1918 - 69622 VILLEURBANNE Cedex / France.

Lesdites souches déposées sont également décrites dans la publication de la demande internationale PCT WO2008/043969.

De tels protozoaires pourront donc être utilisés dans des agents désinfectants, en particulier destinés à l'élimination des amibes *Naegleria fowleri* et à lutter contre la prolifération et la contamination par *Naegleria fowleri.*

D'autre part, l'invention utilise un agent désinfectant contenant des protozoaires de l'espèce Willaertia magna correspondant à la souche déposée sous le numéro PTA 7824 à l'ATCC ou à la souche déposée sous le numéro PTA 7825 à l'ATCC. De façon avantageuse, l'agent désinfectant se présente sous la forme d'une solution ou d'une suspension aqueuse, par exemple dans de l'eau distillée. L'agent désinfectant pourra se présenter sous une forme pulvérisable, par exemple en aérosol ou tout autre moyen d'application.

L'activité des protozoaires de l'espèce Willaertia magna correspondant à la souche déposée sous le numéro PTA 7824 à l'ATCC ou à la souche déposée sous le numéro PTA 7825 à l'ATCC, inhibant la prolifération de *Naegleria fowleri* a été mise en évidence par les inventeurs en comparant la réplication de *Naegleria fowleri* en présence et en absence des protozoaires de l'espèce Willaertia magna correspondant à la souche déposée sous le numéro PTA 7824 à l'ATCC ou à la souche déposée sous le numéro PTA 7825 à l'ATCC.

Les inventeurs ont également démontré le caractère unique de cette inhibition de la croissance de *Naegleria fowleri* par des protozoaires de l'espèce Willaertia magna correspondant à la souche déposée sous le numéro PTA 7824 à l'ATCC ou à la souche déposée sous le numéro PTA 7825 à l'ATCC en utilisant une autre espèce d'amibe libre *Acanthamoeba* comme souche contrôle ne provoquant aucune inhibition de la croissance de *Naegleria fowleri.*

Etant donné l'absence de traitement curatif ou prophylactique du risque *Naegleria fowleri* pour l'homme d'un taux de mortalité de plus de 95% en moins d'une semaine, cette invention est une avancée scientifique majeure pour lutter contre ce fléau amibien avec un impact neutre sur l'environnement et sur l'homme. En effet, la souche *Willaertia magna* est actuellement reconnue sans classe ni catégorie de danger, de mentions de danger ou de conseil de prudence selon le règlement CE n°1271/2008 à l'inverse de la monochloramine, actuellement utilisée.

De plus, la présente invention vise l'utilisation de l'agent désinfectant comme biocide sur les *Naegleria.*

Les exemples ci-après permettent d'illustrer l'invention mais n'ont aucun caractère limitatif.

### 1. MATERIEL ET METHODES

### 1.1. Souches utilisées:

- ***Amibes*** : les souches utilisées appartiennent à trois espèces amibiennes différentes :
   ∘ *Naegleria fowleri (ATCC 30809)*
   ∘ *Acanthamoeba castellanii (ATCC 30010)*
   ∘ *Willaertia magna* (souches déposées à ATCC sous les n° PTA 7824 et PTA 7825).

Ces trois souches sont cultivées axéniquement, en présence de 10% de sérum de veau foetal, sur milieu SCGYEM (Serum Casein Glucose Yeast Extract Medium), réparti en tubes GREINER™ à raison de 3ml par tube. En entretien, les formes végétatives sont repiquées tous les 8-9 jours. Pour les co-cultures, on utilise des repiquages de 3 à 4 jours de manière à disposer de trophozoïtes en pleine phase de croissance exponentielle.

Le milieu SCGYEM est obtenu comme suit :

| | | |
|---|---|---|
| 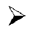 | Caséine (MERCK 1.02244.010) | 10 g |
| 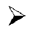 | Na₂HPO₄ | 1,325 g |
| 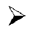 | KH₂PO₄ | 0,8 g |
| 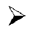 | Glucose | 2,5 g |
| 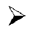 | Extrait de levure (DIFCO 0127-17-9) | 5 g |
| 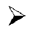 | Eau distillée | 900 ml |
| 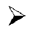 | Sérum de veau foetal | 100 ml |

Aux 900 ml d'eau distillée, sont ajoutés 2,5 ml de NaOH (1N), puis Na₂HPO₄ et KH₂PO₄. Après avoir chauffé légèrement sur plaque chauffante, la caséine est ajoutée progressivement sous agitation magnétique. Après dissolution de la caséine, le glucose et l'extrait de levure sont incorporés.

Après dissolution complète, le milieu est filtré successivement sur fibre de verre (SARTORIUS SM 6513400), puis sur membrane de 1µm (WHATMAN 7190 004). Le milieu est ensuite aliquoté en flacons de verre. Les flacons sont stérilisés à l'autoclave 20 minutes à 120°C. Avant l'utilisation définitive et répartition du milieu, le sérum de veau foetal à raison de 10% du volume final est ajouté stérilement sous hotte à flux laminaire.

### 1.2. Préparation des suspensions mères amibiennes Willaertia magna et Naegleria fowleri:

La préparation de chaque suspension amibienne initiale (suspension mère) s'effectue à partir de 4 flasques (T 175 ml) de culture axénique de chaque amibe cultivée en milieu SCGYEM. Ces suspensions amibiennes mères de *Naegleria fowleri, Acanthamoeba* et *Willaertia magna* sont récoltées en fin de phase de croissance exponentielle qui est généralement obtenue 4 à 5 jours après le démarrage de la culture.

Afin d'augmenter la concentration amibienne récoltée, les flasques sont passées sur un bain de glace pendant 5 à 10 minutes, puis agitées manuellement afin de collecter le plus d'amibes possible.

Le contenu de ces 4 flasques est réuni pour énumérer la suspension amibienne obtenue sur une cellule de THOMA ([C]/ml).
Les suspensions amibiennes sont transférées dans un tube de 50 ml de type FALCON® afin d'éliminer le milieu de culture SCGYEM par centrifugation à 1500g pendant 10 minutes. Les culots amibiens issus de la première centrifugation sont rincés deux fois par lavage à l'eau distillée stérile et centrifugés à 1500g pendant 10 minutes. A l'issue des 2 lavages, le culot final est repris dans un volume d'eau stérile de 40 ml.

### 1.3. Mise en évidence de l'effet biocide des Willaertia magna sur les Naegleria fowleri (souche ATCC 30809) :

Les co-cultures amibiennes sont réalisées dans des flasques T 25 ml contenant 10 ml de milieu PAS (Page's Amoeba Saline) stérile. L'ensemencement des flasques de co-culture amibiennes se fait à raison de 1 × 10⁵ *Willaertia magna*/ml et 1 × 10⁵ *Naegleria fowleri*/ml à partir des suspensions amibiennes axénique préalablement dénombrées sur un hématimètre de Malassez. L'infestation des *Willaertia magna* par *Naegleria fowleri* est réalisée en fixant un ratio *Naegleria fowleri* / *Willaertia magna* de 1. Les flasques T25 ml sont mises en incubation à l'étuve à 30°C.

Le milieu PAS est obtenu comme suit :

| Solution 1: | Quantité pour 500ml d'H₂O | Fournisseur | [M] |
|---|---|---|---|
| NaCl (Poids moléculaire:58.44) | 12.0 g | Fisher S271-3 | 0.41 |
| MgSO₄.7H₂O (Poids moléculaire eptahydrate: 246.47) | 0.40 g | Sigma 63138 (eptahydrate) | 0.0032 |
| CaCl₂.6H₂O (Poids moléculaire: 219.08) | 0.60 g | Sigma 442909-1kg | 0.0055 |

| Solution 2: | | | |
|---|---|---|---|
| Na₂HPO₄ (Poids moléculaire anhydre:141.96) | 14.20 g | Fluka 71629-100g | 0.2 |
| KH₂PO₄ (Poids moléculaire anhydre: 136.09) | 13.60 g | Sigma P5655 | 0.2 |

Filtrer stérilement les solutions 1 et 2 à l'aide d'un filtre 0.22 µM. Afin d'obtenir 1 litre de milieu PAS: ajouter 5.0 ml de la solution 1 et 5.0 ml de solution 2 puis ajuster le volume à 1 litre avec de l'eau distillée stérile.

Les monocultures amibiennes témoins *Willaertia magna* et *Naegleria fowleri* sont séparément cultivées dans des flasques T 25 ml dans 10 ml de milieu PAS stérile. L'ensemencement des flasques se fait à raison de 1 × 10⁵ *Willaertia magna*/ml pour le témoin *Willaertia magna* et 1 × 10⁵ *Naegleria fowleri*/ml pour le témoin *Naegleria fowleri* à partir des suspensions amibiennes axénique préalablement dénombrées sur un hématimètre de Malassez. Les flasques T25 ml témoins sont mises en incubation à l'étuve à 30°C.

Chaque condition est réalisée en triplicata. Chaque comptage sur cellule de Mallassez est répété 5 fois. L'expérience a été renouvelée trois (3) fois sur une période de 3 mois.

Les devenirs des amibes *Naegleria fowleri* et *Willaertia magna* en co-culture et dans les flasques témoins sont déterminés de la façon suivante :
Les concentrations amibiennes sont suivies pendant 120 heures après l'infestation par *Naegleria fowleri.* A chaque intervalle de temps (à 3 heures puis toutes les 24 heures), les flasques de co-culture et des témoins sont prélevés et examinés à la fois du point de vue de la croissance cellulaire des deux amibes et de leur état morphologique et dynamique. Pour chaque flasque examinée:
- La numération des amibes s'effectue directement sur cellule de Malassez.
- A 120 heures, le faible taux d'amibes *Naegleria fowleri* ne permet plus un comptage fiable sur cellule de Mallassez d'où l'utilisation conjointe de la méthode NPP (Nombre le Plus Probable). Cette méthode très utilisée pour le comptage amibien a l'avantage d'être plus précise mais également de discriminer les amibes intègres et vivantes des amibes intègres mais mortes.

### 2. RESULTATS

Les **Figures 1, 2** **et** **3** montrent des expériences de co-culture (*Willaertia magna* / *Naegleria fowleri*) et de monocultures témoins d'amibes.

Elles montrent l'évolution spontanée des populations respectives d'amibes *Willaertia magna* et *Naegleria fowleri* après mise en co-culture à un ratio *Willaertia*/*Naegleria* initial de 1 versus l'évolution des populations respectives d'amibes en monoculture.

Les **Figures 4a et 4b** montrent l'état physiologique des amibes *Naegleria fowleri* au cours du temps en présence d'amibes *Willaertia magna.* La figure 4a correspond à des images de la dégradation physiologique rapide des *Naegleria fowleri* en co-cultures avec *Willaertia magna.* La figure 4b correspond à des image illustrant le phénomène du "baiser de la mort" d'une *Willaertia magna* sur une *Naegleria fowleri.*

La **Figure 5** montre une expérience de co-culture (*Acanthamoeba castelanii* / *Naegleria fowleri*) et de monocultures témoins d'amibes.

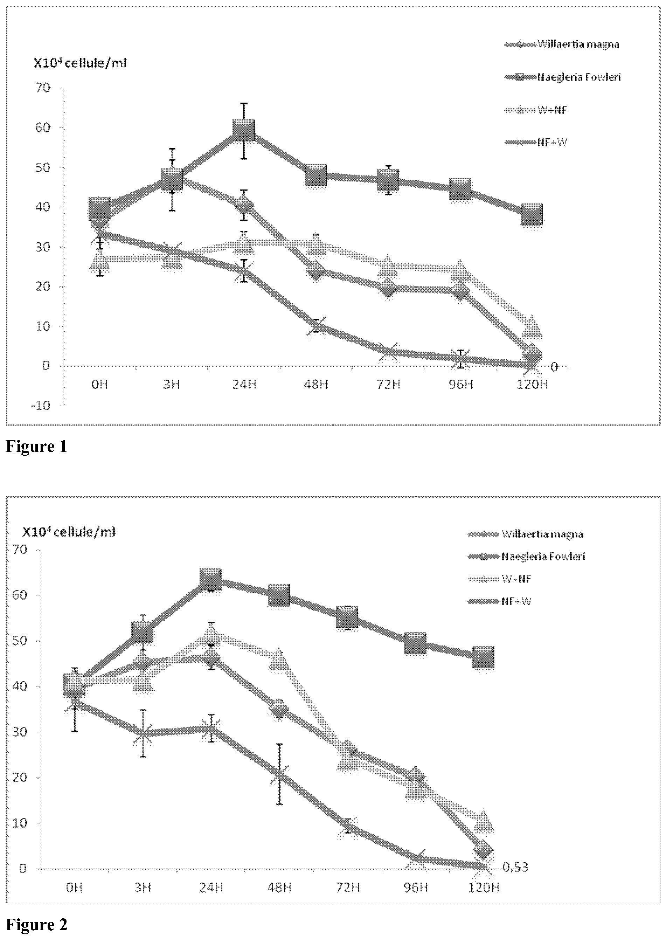

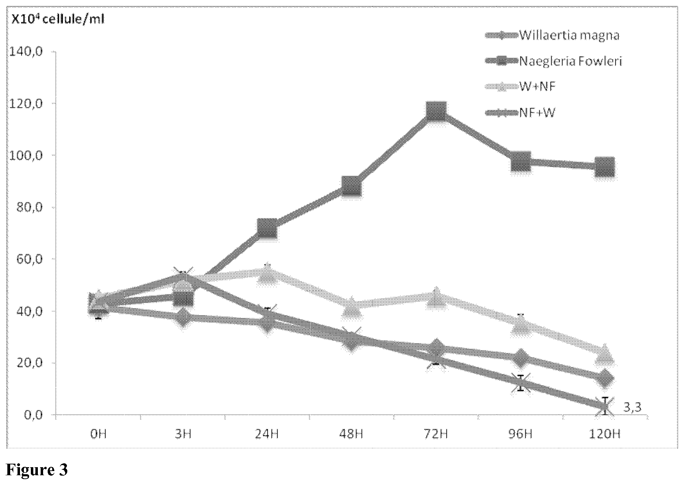

Sur les figures 1, 2 et 3, la courbe avec des points en forme de losange (◆) nommée *Willaertia magna* décrit la concentration mesurée de *Willaertia magna* seule en culture dans un milieu PAS stérile.

La courbe avec des points en forme de carrée (■) nommée *Naegleria fowleri* décrit la concentration mesurée de *Naegleria fowleri* seule en culture dans un milieu PAS stérile.

La courbe avec des points en forme de triangle (Δ) nommée *W +NF* décrit la concentration mesurée de *Willaertia magna* en co-culture avec *Naegleria fowleri* dans un milieu PAS stérile.

La courbe avec des points en forme de croix (X) nommée *NF* + *W* décrit la concentration mesurée de *Naegleria fowleri* en co-culture avec *Willaertia magna* dans un milieu PAS stérile.

Les données sont exprimées en concentration de cellules intègres par millilitres (ml), comptées sur cellules de Mallassez.

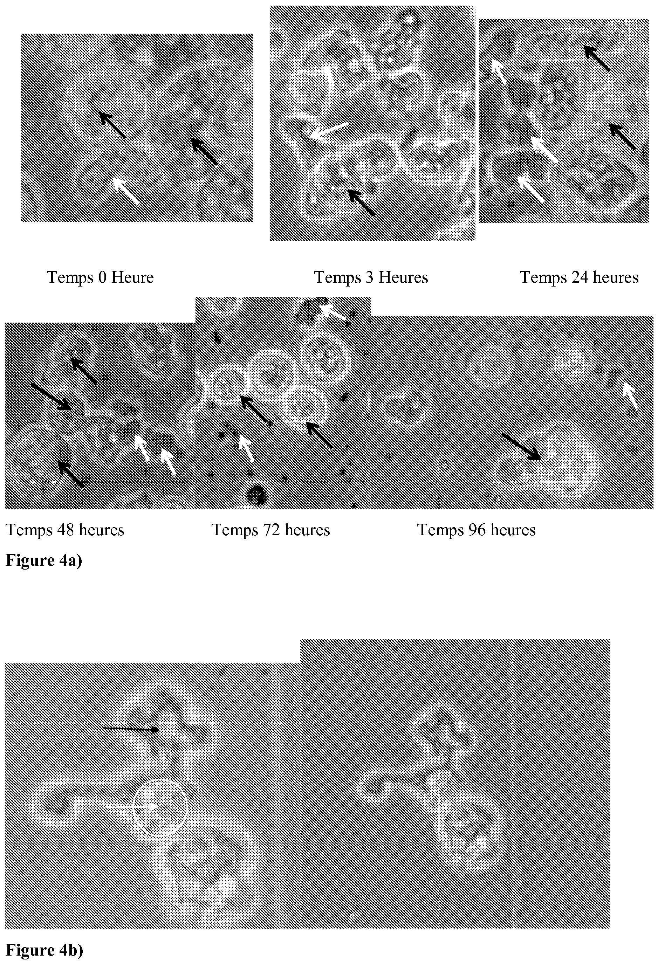

Sur la figure 4a, les flèches noires indiquent la présence de *Willaertia magna* et les flèches blanches la présence de *Naegleria fowleri* entières ou en morceaux.

Sur la figure 4b, la flèche noire indique la présence de *Willaertia magna* et la flèche blanche de *Naegleria fowleri.* Le rond blanc matérialise les contours de la cellule de *Naegleria fowleri.* Ce phénomène de "baiser de la mort" a été décrit dans la littérature (Berke G. Source Department of Cell Biology, 1995) comme un contact permettant le passage de granzyme conduisant à l'apoptose de la cellule cible.

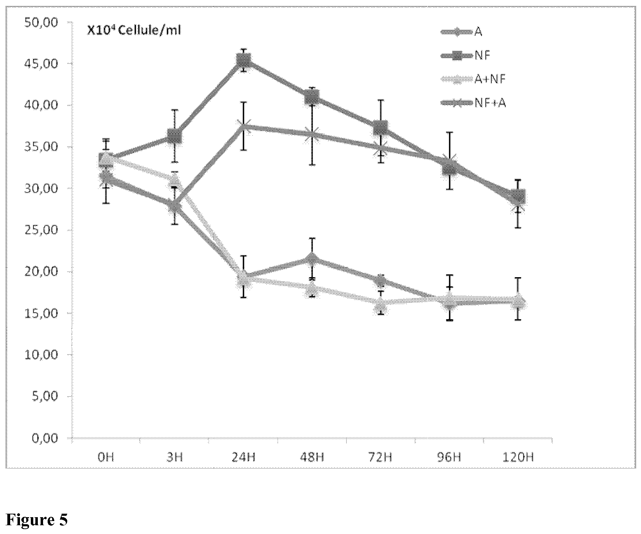

Sur la figure 5, la courbe A composée de points en losange (◆) représente une mono-culture *d'Acanthamoeba castellanii.*

La courbe A + NF composée de points en triangle (Δ) représente la concentration *d'Acanthamoeba castellanii* en co-culture *avec Naegleria fowleri.*

La courbe NF composée de points en carré (■) représente une mono-culture de *Naegleria fowleri.*

La courbe NF +A composée de points en croix (X) représente la concentration de *Naegleria fowleri* en co-culture *avec d'Acanthamoeba castellanii.*

### 2. 1. Willaertia magna inhibe totalement la croissance de Naegleria fowleri.

Comme indiqué par les courbes en croix des **figures 1, 2** **et** **3** représentant l'évolution des *Naegleria fowleri* en co-culture avec des *Willaertia magna,* très rapidement vers 24 heures un décrochage intervient dans la courbe de population des *Naegleria fowleri.* Cette décroissance de la population des *Naegleria fowleri* est observée uniquement en présence de *Willaertia magna.* Les courbes témoins (courbes avec des points carrés) de *Naegleria fowleri* en monoculture dans les expériences 1, 2 et 3 **(****figures 1, 2** **et** **3****)** à 24 heures sont toutes en croissance. D'ailleurs, le taux de *Naegleria fowleri* en monoculture double quasiment à 24 heures.

Fait remarquable, l'observation microscopique, confirme un changement morphologique cruciale des *Naegleria fowleri* en co-culture avec des *Willaertia magna* dès 24 heures (figure 4). L'état physiologique de la cellule *Naegleria fowleri* passe d'une forme trophozoïte étalée à une forme plus ronde stressée, mais non encore apparentée à la forme kystique. L'intérieur de la cellule de *Naegleria fowleri* devient opaque, masquant les vacuoles internes, signe de croissance des amibes. Les cellules de *Naegleria fowleri* en présence de *Willaertia magna* ont une taille inférieure de moitié à celle en monoculture dès 3 heures de co-culture (figure 4a). Le mode de prédation des amibes *Willaertia magna* est basé sur la phagocytose. La figure 4b démontre dans les 24 premières heures un phénomène d'inhibition proche « du baiser de la mort ». La phagocytose de *Naegleria fowleri* par *Willaertia magna* intervient bien, mais seulement après 25 heures de co-culture corroboré par la présence de *Willaertia magna* contenant dans ces phagosomes des *Naegleria fowleri* (données non reportées).

L'ensemble des expériences conduites démontre un effet maximum d'inhibition avec une élimination complète des *Naegleria fowleri* en 120 heures. La lecture en cellule de Mallassez à 120 heures dénombre les cellules de *Naegleria fowleri* présentes et intègres mais il n'est pas possible de discriminer par ce comptage, les cellules mortes des cellules vivantes. Une double lecture sur gélose NNA + E.coli permet le comptage des cellules de Naegleria fowleri vivantes uniquement issues de forme trophozoïtes et/ou de formes kystiques.

Le tableau ci-dessous indique la concentration amibiennes (*Naegleria fowleri)* obtenues par lecture sur cellules de Mallassez et celles obtenues par la méthode NPP.

Un aliquot de chaque flasque issue des expériences 1, 2 et 3 (cf. figures 1 à 3) de co-culture *Willaertia magna* / *Naegleria fowleri* pris à 120 heures post infection est ensemencé sur des géloses NNA recouverte d'un tapis d'E-coli.

La détermination de la concentration de chaque espèce est mesurée par le comptage du nombre de fronts repérés par boites, et renseignés dans la table NPP pour en déduire la concentration amibienne.
Nf = *Naegleria fowleri*
W.m = *Willaertia magna*

| | Mesure à 120 heures par la méthode NPP (limite de quantification = 4 Nf/l) | Mesure à 120 heures par comptage sur cellule de Malassez (limite de quantification = 0,2 10⁴ Nf/l) |
|---|---|---|
| Expérience 1 co-culture Nf et W.m (figure 1) | < 201 Nf/l | 0 x 10⁴ Nf/ml |
| Expérience 2 co-culture Nf et W.m (figure 2) | < 201 Nf/l | 0,53 x 10⁴ Nf/ml |
| Expérience 3 co-culture Nf et W.m (figure 3) | < 201 Nf/l | 3,3 x 10⁴ Nf/ml |

Ce tableau confirme l'absence de fronts de *Naegleria fowleri* sur les géloses NNA, indiquant l'absence de *Naegleria fowleri* sous forme kystiques ou trophozoïtes vivantes à 120 heures.

Les inventeurs ont mis en évidence que le caractère inattendu d'un effet biocide des protozoaires de l'espèce Willaertia magna correspondant à la souche déposée sous le numéro PTA 7824 à l'ATCC ou à la souche déposée sous le numéro PTA 7825 à l'ATCC sur le genre *Naegleria* et plus particulièrement sur l'espèce *Naegleria fowleri* n'est pas partagé par d'autres espèces amibiennes. Comme indiquée par la figure 5, une co-culture des genres amibiens *Acanthamoeba castellanii* et *Naegleria fowleri* n'induit aucune incidence sur la concentration respective des deux espèces versus la concentration mesurée de ces espèces en monoculture. Cette figure montre l'absence d'action réciproque d'un autre genre amibien, de type *Acanthamoeba* sur l'évolution de la population de *Naegleria fowleri* lors d'une mise en co-culture de ces deux amibes.

*Willaertia magna* possède bien seule cette caractéristique d'inhibition de la croissance des amibes *Naegleria* et plus particulièrement l'espèce *Naegleria fowleri.*

### Bibliographie

**Bodennec Jacques, Pernin Pierre, Dey Rafick.** Novel method for biologically combating the proliferation of Legionella pneumophila, and novel disinfecting agent containing amoebic protozoa of the Willaertia genus. France Brevet 2906968. 12 10 2006.
Cervantes-Sandoval I, Serrano-Luna J, Garcia-Latorre E, Tsutsumi V, Shibayama M. «Characterization of brain inflammation during primary amoebic meningoencephalitis .» Parasitol Int, 57 (2008): 307 e13.
De Jonckheere. «Origin and evolution of the worldwide distributed pathogenic amoeboflagellate Naegleria fowleri.» Infect Genet Evol. 11, n°(7) (Oct; 2011): 1520-8.
Gautam PL, Sharma S, Puri S, Kumar R, Midha V, Bansal R. «A rare case of survival from primary amebic meningoencephalitis..» Indian J Crit Care Med. 16, n° (1) (Jan. 2012): 34-6..
Goudot S, Herbelin P, Mathieu L, Soreau S, Banas s, Jorand F. «Growth dynamic of Naegleria fowleri in a microbial freshwater biofilm.» Water res. 46, n° (13) (Sep. 2012): 3958-66.
Huang SW, Hsu BM. «Survey of Naegleria and its resisting bacteria-Legionella in hot spring water of Taiwan using molecular method..» Parasitol Res. 106, n° (6) (May 2010): 1395-402.
Jamerson M, Remmers K, Cabral G, Marciano-Cabral F. «Survey for the presence of Naegleria fowleri amebae in lake water used to cool reactors at a nuclear power generating plant.» Parasitol Res. 104, n° (5) (Apr; 2009): 969-78.
Kemble SK, Lynfield R, DeVries AS et al. « Fatal Naegleria fowleri infection acquired in Minnesota: possible expanded range of a deadly thermophilic organism .» Clin Infect Dis 54 (2012): 805-809.
Martinez AJ, Visvesvara GS. «Free-living, amphizoic and opportunistic amebas.» Brain Pathol. 7, n° (1) (Jan; 1997): 583-98. Review.
S.A. Huws, A.J. McBain and P. Gilbert. «Protozoan grazing and its impact upon population dynamics in biofilm communities .» Journal of Applied Microbiology 2005, 98, 238-244 98 (2005): 238-244.
Su MY, Lee Shyu LY, Lin WC, Hsiao PC, Wang CP, Ji DD, Chen KM, Lai SC. «A fatal case of Naegleria fowleri menigoencephalitis in Taiwan.» Korean J parasitol. 51, n° (2) (April 2013): 203-206.
TW, Heggie. «Swimming with death: Naegleria fowleri infections in recreational waters .» Travel Med Infect Dis 8 (2010): 201-206.
Visvesvara GS, Moura H, Schuster FL. «Pathogenic and opportunistic free-living amoebae: Acanthamoeba spp., Balamuthia mandrillaris, Naegleria fowleri, and Sappinia diploidea.» FEMS Immunol Med Microbiol 50 (2007): 1-26.

## Revendications

1. Procédé de lutte contre la prolifération d'amibes du genre *Naegleria,* à l'exception des méthodes de traitement appliquées au corps humain ou animal, **caractérisé en ce qu'**il utilise des protozoaires de l'espèce *Willaertia magna* correspondant à la souche déposée sous le numéro **PTA 7824** à l'ATCC ou à la souche déposée sous le numéro **PTA 7825** à l'ATCC.

2. Procédé selon la revendication 1, pour lutter contre la prolifération de l'espèce *Naegleria fowleri.*

3. Procédé selon la revendication 1 ou 2, **caractérisé en ce que** l'on traite un flux gazeux ou liquide, ou une surface solide avec des protozoaires de l'espèce *Willaertia magna.*

4. Procédé selon l'une des revendications 1 à 3, **caractérisé en ce qu'**il est mis en oeuvre pour la désinfection des réseaux de distribution des eaux sanitaires ou des eaux industrielles, des circuits de refroidissement des installations industrielles ou nucléaires, ou des réseaux de climatisation.

5. Procédé selon l'une des revendications 1 à 3, **caractérisé en ce qu'**il est mis en oeuvre pour lutter contre la formation de biofilms dans les canalisations d'eau, les surfaces en contact ou non avec des denrées alimentaires humaines ou animales.

6. Utilisation d'un agent désinfectant contenant des protozoaires de l'espèce *Willaertia magna,* comme biocide sur les *Naegleria,* à l'exception d'une utilisation pour le traitement appliqué au corps humain ou animal, **caractérisée en ce que** les protozoaires correspondent à la souche déposée sous le numéro **PTA 7824** à l'ATCC ou à la souche déposée sous le numéro **PTA 7825** à l'ATCC.

7. Utilisation selon la revendication 6, **caractérisée en ce que** l'agent désinfectant se présente sous la forme d'une solution ou d'une suspension aqueuse.

## Patentansprüche

1. Verfahren zur Kontrolle der Vermehrung von Amöben der Gattung *Naegleria,* mit Ausnahme von Behandlungsmethoden, die auf den menschlichen oder tierischen Körper angewendet werden, **dadurch gekennzeichnet, dass** es Protozoen der Art *Willaertia magna* verwendet, die dem Stamm entsprechen, der unter der Nummer **PTA 7824 beim** ATCC hinterlegt ist, oder dem Stamm, der unter der Nummer **PTA 7825 beim** ATCC hinterlegt ist.

2. Verfahren nach Anspruch 1 zur Kontrolle der Proliferation der Art *Naegleria fowleri.*

3. Verfahren nach Anspruch 1 oder 2, **dadurch gekennzeichnet, dass** ein gasförmiger oder flüssiger Strom oder eine feste Oberfläche mit Protozoen der Art *Willaertia magna* behandelt wird.

4. Verfahren nach einem der Ansprüche 1 bis 3, **dadurch gekennzeichnet, dass** es zur Desinfektion von Haus- oder Industriewasserversorgungsnetzen, Kühlkreisläufen von Industrie- oder Kernanlagen oder Klimatisierungsnetzen verwendet wird.

5. Verfahren nach einem der Ansprüche 1 bis 3, **dadurch gekennzeichnet, dass** es zur Verhinderung der Bildung von Biofilmen in Wasserleitungen, Oberflächen, die mit menschlichen oder tierischen Lebensmitteln in Berührung kommen oder nicht, verwendet wird.

6. Verwendung eines Desinfektionsmittels, das Protozoen der Art *Willaertia magna* enthält, als Biozid auf *Naegleria,* mit Ausnahme einer Verwendung für die Behandlung des menschlichen oder tierischen Körpers, **dadurch gekennzeichnet, dass** die Protozoen dem Stamm entsprechen, der unter der Nummer **PTA 7824 beim** ATCC hinterlegt ist, oder dem Stamm, der unter der Nummer **PTA 7825 beim** ATCC hinterlegt ist.

7. Verwendung nach Anspruch 6, **dadurch gekennzeichnet, dass** das Desinfektionsmittel in Form einer wässrigen Lösung oder Suspension vorliegt.

## Claims

1. A process for combatting the proliferation of the *Naegleria* genus, with the exception of the treatment methods applied to the human or animal body, **characterized in that** it uses protozoa of the species *Willaertia magna* corresponding to the strain deposited under number **PTA 7824** at the ATCC or to the strain deposited under number **PTA 7825** at the ATCC.

2. The process according to claim 1, for combatting the proliferation of *Naegleria fowleri* species.

3. The process according to claim 1 or 2, **characterized in that** a gaseous flow or liquid, or solid surface is treated with protozoa of Willaertia magna.

4. The process as claimed in one of claims 1 to 3, **characterized in that** it is implemented for the disinfection of sanitation water or industrial water distribution networks, cooling circuits of industrial or nuclear plants, or air-conditioning networks.

5. The process as claimed in one of claims 1 to 3, **characterized in that** it is implemented for controlling the formation of biofilms in water pipes, or surfaces which may or may not be in contact with human or animal foodstuffs.

6. Use of a disinfecting agent containing protozoa of the *Willaertia* magna, as a biocide on *Naegleria,* with the exception of the treatment methods applied to the human or animal body, **characterized in that** the protozoa correspond to the strain deposited under number **PTA 7824** at the ATCC or to the strain deposited under number **PTA 7825** at the ATCC.

7. The use of Claim 6, **characterized in that** the disinfecting agent is presented under the form of a solution or an aqueous suspension.
